# EUROPEAN PATENT SPECIFICATION

(11) **EP 3 965 840 B1**
(45) Date of publication and mention of the grant of the patent: **26.08.2026**
(21) Application number: 20805363.7
(22) Date of filing: 08.05.2020
(51) Int. Cl.: A61L 27/36, A61L 27/54, A61L 27/38

(54) **MINIMAL PROCESSING METHOD FOR DECELLULARIZATION OF TISSUES**
MINIMALVERARBEITUNGSVERFAHREN ZUR DEZELLULARISIERUNG VON GEWEBEN
PROCÉDÉ DE TRAITEMENT MINIMAL POUR LA DÉCELLULARISATION DE TISSUS

(30) Priority: 10.05.2019 US 201962845959 P
(43) Date of publication of application: 16.03.2022
(73) Proprietor: Wake Forest University Health Sciences, Winston-Salem, NC 27157 (US)
(72) Inventor: ORLANDO, Giuseppe, Winston-Salem, North Carolina 27106 (US); TAMBURRINI, Riccardo, Winston-Salem, North Carolina 27104 (US)
(74) Representative: Murgitroyd & Company
(86) International application number: PCT/US2020/031995
(87) International publication number: WO 2020/231768

(56) References cited:
- US-A1- 2008 306 610
- US-A1- 2011 045 566
- US-A1- 2011 151 011
- US-A1- 2018 282 699
- NICOLYNN E DAVIS ET AL: "Enhanced function of pancreatic islets co-encapsulated with ECM proteins and mesenchymal stromal cells in a silk hydrogel", BIOMATERIALS, ELSEVIER, AMSTERDAM, NL, vol. 33, no. 28, 14 June 2012 (2012-06-14), pages 6691 - 6697, XP028428381, ISSN: 0142-9612, [retrieved on 20120619], DOI: 10.1016/J.BIOMATERIALS.2012.06.015
- TONDREAU ET AL.: "Mechanical properties of endothelialized fibroblast-derived vascular scaffolds stimulated in a bioreactor", ACTA BIOMATERIALIA, May 2015 (2015-05-01), pages 176 - 185, XP055762148
- "Hypertonic and Hypotonic Environments", Retrieved from the Internet <URL:https://www2.hawaii.edu/~johnb/micro/m140/syllabus/week/handouts/m140.9.2.html> [retrieved on 20200722]

## Description

### BACKGROUND

Islet transplantation (ITX) is a treatment offered to patients affected by type 1 diabetes as an alternative to whole pancreas transplantation. However, despite ITX's efficacy in restoring insulin secretion, normalizing glucose homeostasis and preventing complications of the disease, its clinical applicability is limited by a lack of mid- and long-term efficacy. In fact, while the half-life of whole pancreas transplantation is 12-14 years, only few patients who have received ITX will be insulin-free 5 years after the transplant.

The underperformance of ITX may be due to the stress that islets are subjected to during their isolation process. Current ITX protocols rely on the isolation of islets from the pancreas through a harsh process that involves enzymatic digestion of the organ (Ricordi's method), whereby the pancreas is demolished, its connections to the body are lost with consequent lack of oxygen and nutrients supply, and islets are irreversibly damaged.

Islet damage may also be due to the destruction of the extracellular matrix (ECM), which represents the 3D framework of the islet niche *(see* Frantz et al., "The extracellular matrix at a glance," Journal of Cell Science 123, 4195-4200, 2010) and whose loss has been associated with the progression of the diabetes. Bogdani et al., "Extracellular Matrix Components in the Pathogenesis of Type 1 Diabetes," Curr Diab Rep 14(12), 552, 2014. Moreover, as ECM signaling to islets is critical for islet function, destruction of the islet niche - whose scaffold is represented by the ECM - may contribute to the limited graft survival observed in clinical ITX. Therefore, reconstitution of the islet niche could aid in transplanted islets functioning better and living longer.

ECM scaffolds derived from mammalian pancreas through a process called decellularization offer a valuable tool to achieve this goal. Peloso et al., "The Human Pancreas as a Source of Protolerogenic Extracellular Matrix Scaffold for a New-Generation Bioartificial Endocrine Pancreas," Annals of Surgery 264(1), 169-179, 2016. Such decellularization, however, is usually obtained using detergent-based solutions, which can be harsh to the innate ECM, causing significant damage and clearance of the molecular fingerprint of the ECM, the matrisome.

Improved methods to obtain ECM scaffolds from tissues that can maintain the matrisome are needed.

US 2018/0282699 describes microenvironments for self-assembly of islet organoids from stem cells differentiation, including a gel solution made by decellularized tissue-specific extracellular matrix (dpECM).

### SUMMARY

Provided herein are improved methods of decellularizing pancreas and other tissues. The methods are more gentle, may be detergent-free, and water-based, and allow for the production of acellular scaffolds whereby the matrisome (i.e., ECM and associated proteins/factors) is better preserved. The methods also enable decellularization without the use of chemicals that may be harmful to the ECM.

A method of decellularizing human tissue or porcine tissue (e.g., pancreas, kidney, liver or muscle tissue) is provided in claim 1, said method comprises providing the tissue; incubating the tissue in a hypoosmotic solution for a time of from 12 to 24, 36 or 48 hours; and incubating the tissue with DNase, to thereby decellularize the tissue. The method does not comprise incubating the tissue with a detergent, and the method is protease-free.. In some embodiments, the method is toxin-free. In some embodiments, the hypoosmotic solution consists essentially of or consists of water.

The method comprises incubating the tissue with DNase to digest the DNA and/or other cellular materials.

In some embodiments, the method comprises: (c) incubating the tissue in a composition comprising an enzyme (e.g., comprising DNAse) for a time sufficient to digest nucleic acids (e.g., for a time of from 2 to 8, 10 or 12 hours); then (d) incubating the tissue in a solution that deactivates the enzyme (e.g., TRIS in water to chelate MgCl, deactivating DNAse) (e.g., for a time of from 8 to 24, 30 or 36 hours); and then (e) incubating the tissue in a second hypoosmotic solution (e.g., consisting essentially of or consisting of water) for a time of from 12 to 24, 36 or 48 hours.

In some embodiments, one or more of the incubating steps is carried out with mechanical agitation of the tissue (e.g., with a shaker at about 50, 100, 150, 200, 250 or 300 rpm). In some embodiments, each of the incubating steps are carried out with mechanical agitation of the tissue (e.g., with a shaker at about 50, 100, 150, 200, 250 or 300 rpm).

In some embodiments, the tissue is pancreas tissue.

In some embodiments, one or more of the incubating steps (b), (d), and (e) are carried out at a temperature of from 2 to 15 degrees Celsius (e.g., about 4 degrees Celsius). In some embodiments, the incubating step (c) is carried out at a temperature of from 25 to 40 degrees Celsius (e.g., about 37 degrees Celsius).

In some embodiments, the tissue of the providing step has been disinfected (e.g., by incubation in a disinfecting solution and/or antibiotic solution).

In some embodiments, the method further comprises dehydrating the tissue (e.g., by lyophilization) after the decellularizing. In some embodiments, the method further comprises milling the tissue after the dehydrating to form a powder.

In some embodiments, the method further comprises de-lipidization after the decelluarizing, dehydrating and/or milling (e.g., by incubation with a proteinase such as pepsin).

Also provided is a composition comprising decellularized tissue, which may be produced by a process as taught herein. A composition is provided in claim 9, and that composition comprises less than 100 ng or 50 ng of DNA per mg dry weight (i.e., weight of the ECM composition after water is removed, such as by lyophilizing). and that composition comprises a total collagen content of from 20-40 micrograms per milligram dry weight of the composition. In some embodiments, the composition comprises a glycosoaminoglycan (GAG) content of from 2-10 micrograms per milligram dry weight.

In some embodiments, the composition comprises an elastin content of from 5-25 micrograms per milligram dry weight of the composition.

In some embodiments, the composition comprises one or more (e.g., 2, 3, 4, 5, 6, 7, 8, 9, 10, 11, 12) of the growth factor/growth factor families listed in Table 1.

In some embodiments, the composition comprises a content of growth factors of greater than about 30, 40, 50, or 60% of the total abundance of an extracelluar matrix (ECM) component of the decellularized proteome.

In some embodiments, the composition comprises a content of matrisome proteins of greater than about 70, 75, 80, 85, or 90% of the total abundance of the decellularized proteome, and a content of cellular proteins of less than about 20, 15, 12 or 10% of the total abundance of the decellularized proteome.

In some embodiments, the composition (e.g., powder composition) has an endotoxin concentration of less than 0.5 EU/ml.

Also provided is a cell culture substrate comprising a coating comprising an ECM composition as taught herein (e.g., an ECM composition produced as taught herein), as provided in claim 11, wherein the coating is optionally in the form or a gel or a powder. Further provided is a method for growing cells *in vitro* (e.g., pancreatic, liver, muscle or kidney cells, progenitor cells thereof, or stem cells) comprising the steps of: contacting said cells to a cell culture substrate, wherein said cells adhere to said coating; and growing said cells *in vitro* under conditions conducive to the proliferation of said cells. In some embodiments, the cell culture substrate comprises a decellularized tissue composition that has not been de-lipidized, and wherein the growing is carried out with media comprising a decellularized tissue composition that has been de-lipidized.

Still further provided is a cell culture media comprising an ECM composition as taught herein, wherein the media is optionally serum-free. A method for growing cells *in vitro* is provided in claim 12.

Also, as provided in claim 13, provided herein is a microparticle comprising encapsulated live cells (e.g., pancreatic, liver, muscle or kidney cells, progenitor cells thereof, or stem cells) and the ECM composition as taught herein. In some embodiments, the live cells are pancreatic islet cells, optionally encapsulated in alginate.

Further disclosed but not claimed is a method of treating Type I diabetes in a subject in need thereof comprising administering the microparticle to said subject in a treatment effective amount. Provided in claim 14 is a microparticle as taught herein for use in a method of treating Type I diabetes. Disclosed herein is the preparation of a medicament for treating Type I diabetes.

Also, as provided in claim 15, provided herein is a method of differentiating pancreatic progenitor cells into pancreatic beta cells, said method comprising growing the pancreatic progenitor cell in the presence of the ECM composition as taught herein, optionally wherein the pancreatic beta cells are formed in islet-like clusters that are responsive to glucose.

### BRIEF DESCRIPTION OF THE DRAWINGS

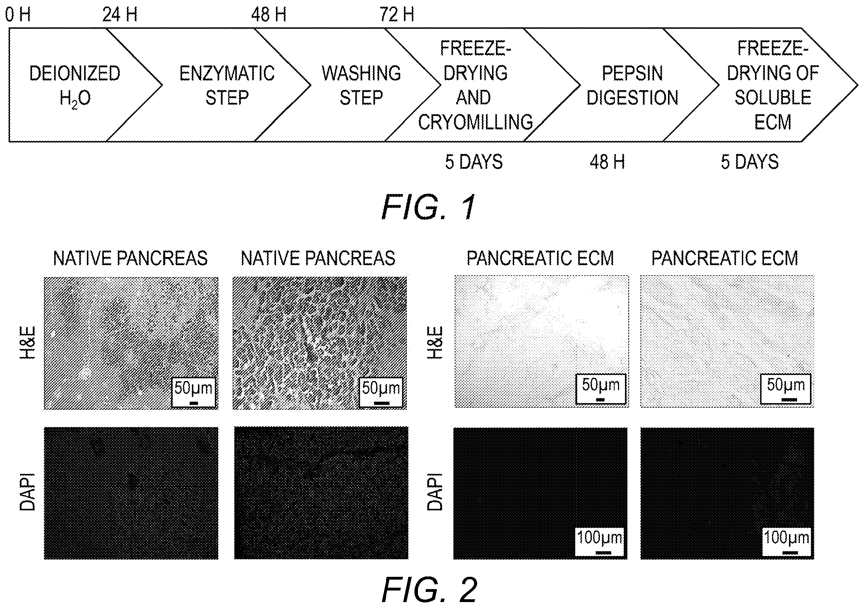

FIG. 1 presents a flow diagram of an example method of decellularizing tissue and producing a soluble ECM composition with minimal processing as taught by the present application.
FIG. 2 presents representative H&E and DAPI histological images of native human pancreas and human pancreatic ECM decellularized with the minimal processing method. H&E panel demonstrated a complete loss of nuclear structures compared to the native pancreatic tissue. DAPI panel demonstrated a complete loss of nuclei compared to the native pancreatic tissue.

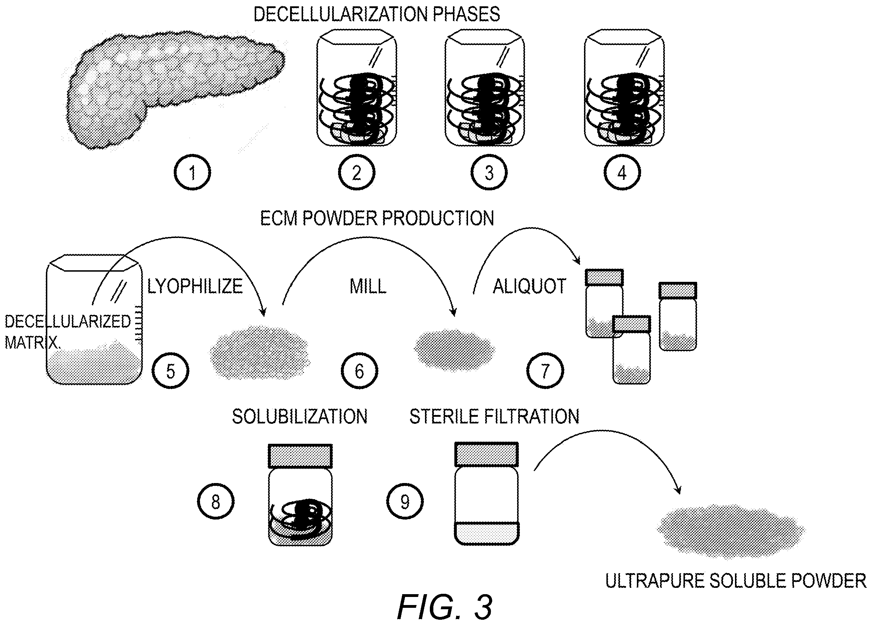

FIG. 3 presents a schematic of steps that may be used to make a soluble powder of a decellularized tissue (pancreas is shown) according to some embodiments.

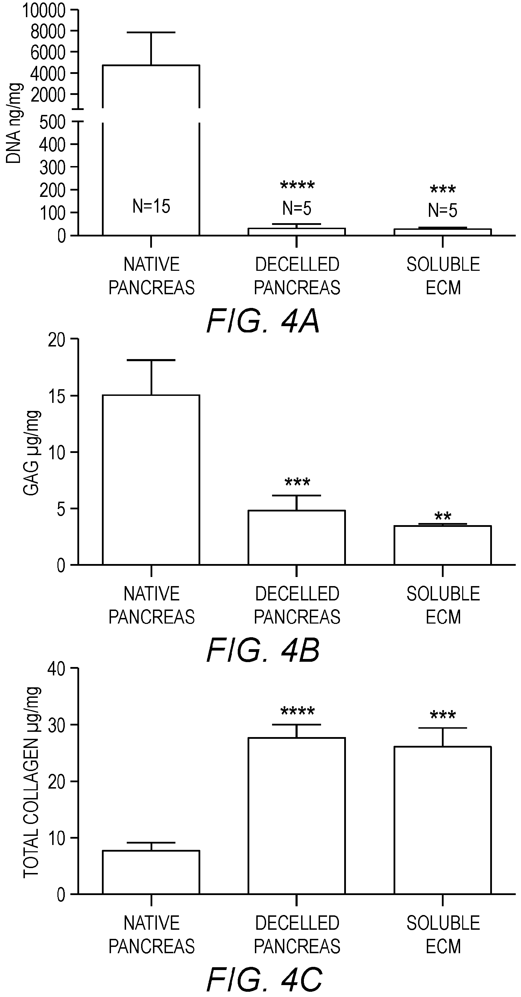

FIGS. 4A-4C present biochemical characterization of native pancreas, acellular pancreatic ECM and soluble pancreatic ECM. FIG. 4A data confirm satisfactory removal of DNA in the acellular pancreas and in the soluble pancreatic ECM. FIG. 4B and FIG. 4C show significant differences in glycosaminoglycans and collagen quantification in the native pancreas compared to the acellular and solubilized ECM. Statistical analysis was performed by the t-test of native vs Decelled pancreas and native vs soluble ECM; ****=p<0.0001;***p<0.001; **p<0.01.

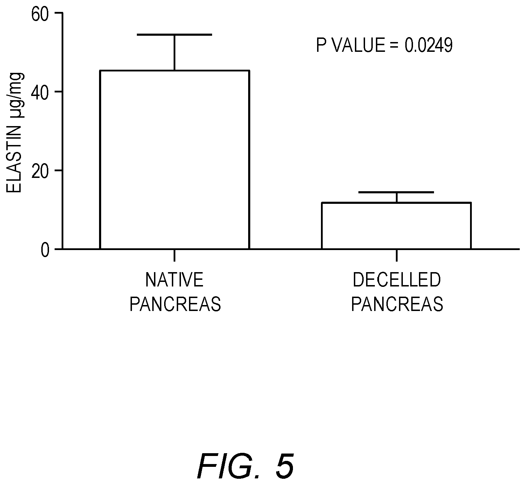

FIG. 5 presents additional characterization data of ECM elastin content of decellularized human pancreas tissue prepared with the minimal processing methods of the present application as compared to native pancreas.

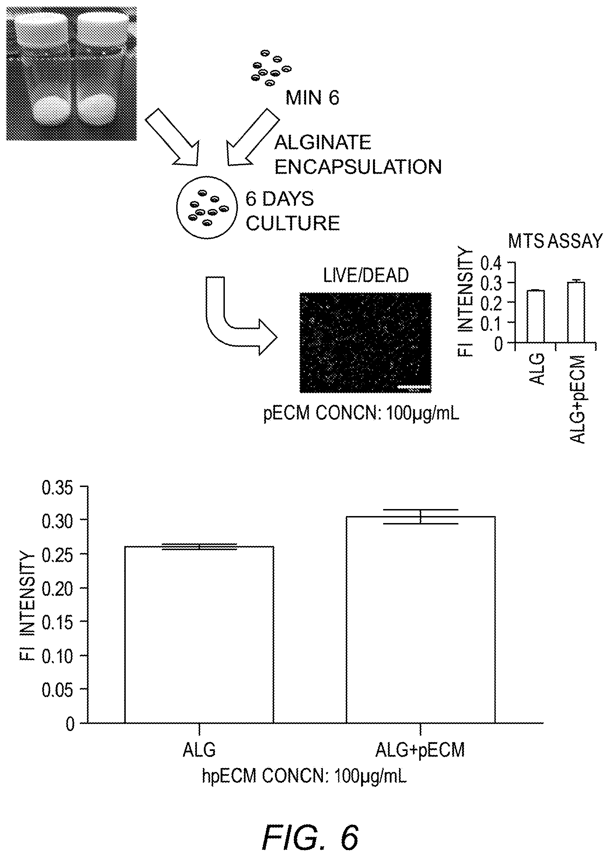

FIG. 6 presents a schematic of the encapsulation of Min6 cells in alginate with and without the soluble ECM powder, and MTS assay data at day 6 of the culture.

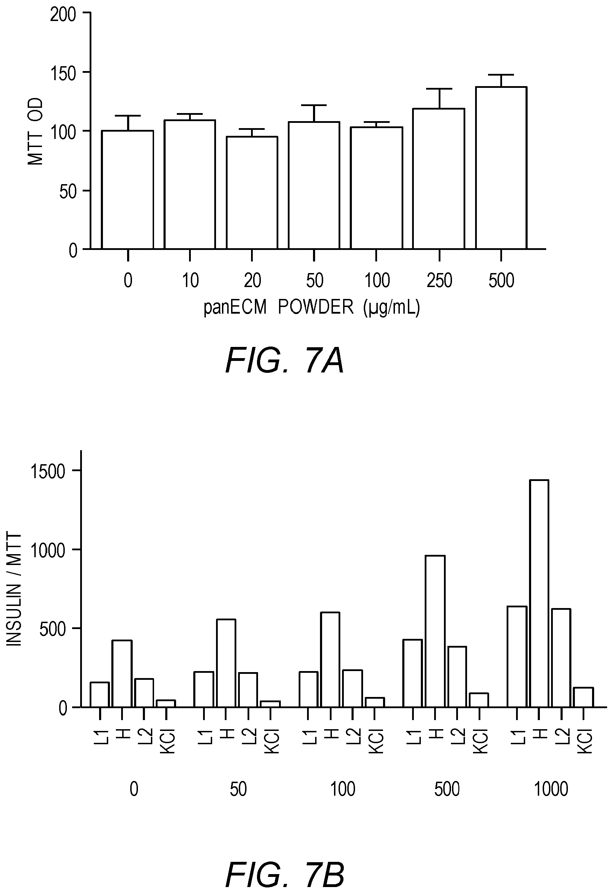

FIGS. 7A-7B presents data from co-culture of Min6 cell monolayer with pancreatic hpECM powder in culture media. FIG. 7A: MIN6 cell viability as a function of powder concentration in the culture media indicated by MTT absorbance (OD) suggests that the hpECM powder is not toxic. FIG. 7B: Min6 cell glucose-stimulated insulin release (GSIR) as a function of powder concentration in the culture media indicated suggesting that the hpECM powder may improve basal insulin secretion.

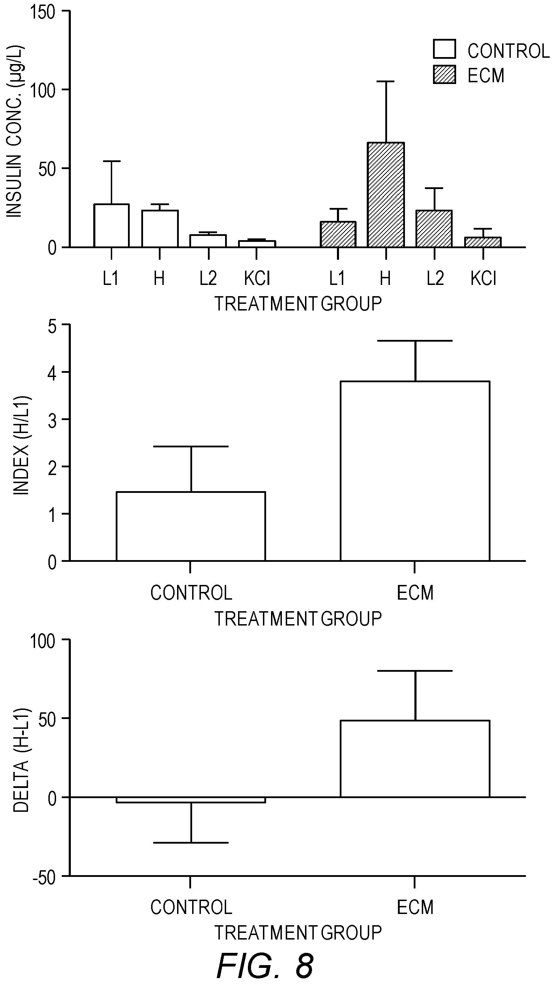

FIG. 8 presents data from culture of murine islets seeded on a plate coated with the pancreatic hpECM powder. Islet function measured through GSIR was improved by hpECM addition to culture substrates, suggesting that the hpECM powder was beneficial for preserving islet function during prolonged *ex vivo* culture.

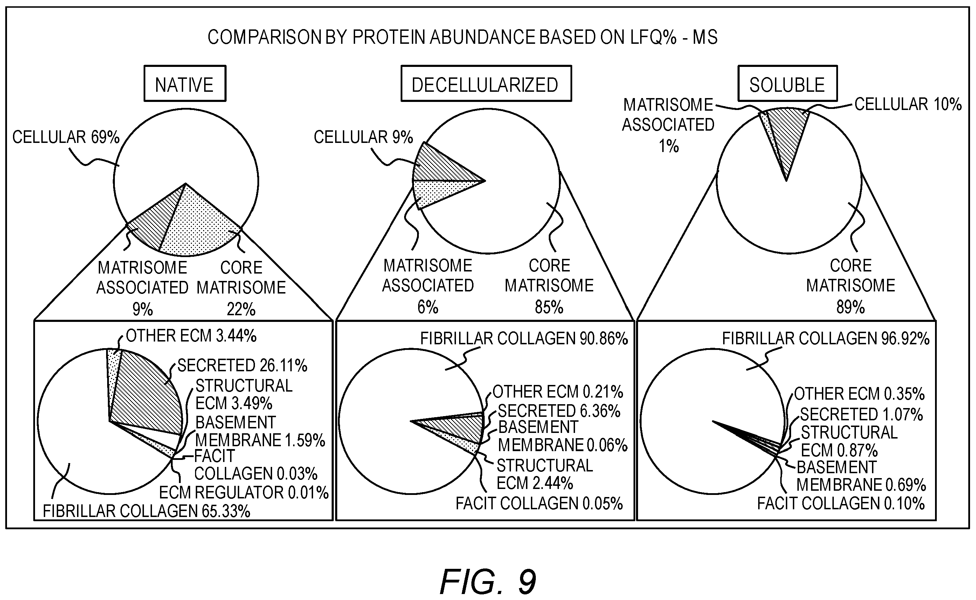

FIG. 9 presents a comparison by protein abundance of native, decellularized and soluble pancreas tissue based on LFQ% mass spectrometry. Matrisomal proteins were found to account for 91% of the total abundance of the decellularized proteome. Even though the DNA content was <50 ng/mg, cellular proteins were still retained, accounting for 9% of abundance. ECM-only analysis again revealed high abundance of Fibrillar Collagen, which had been enriched to 91% of the matrisome. All the other functional categories were found to be diminished.

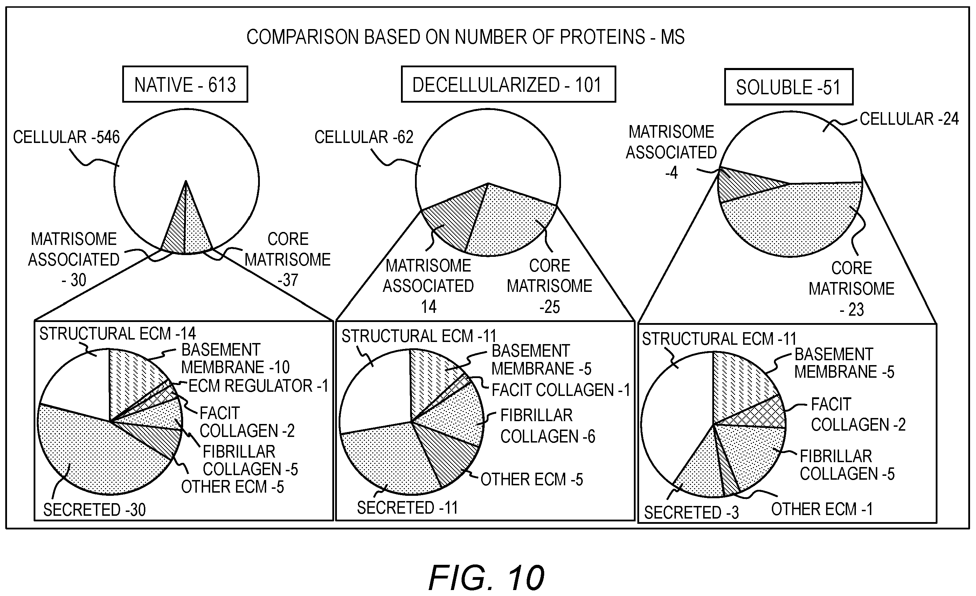

FIG. 10 presents a comparison based on number of proteins of native, decellularized and soluble pancreas tissue using mass spectrometry. Classification on the basis of protein number revealed that 67 proteins formed the native Matrisome, while 546 were found to be cellular proteins. After decellularization, 62 cellular proteins were still retained. A loss of 28 matrisomal proteins was also observed. The soluble ECM was composed of 51 proteins. 27 Matrisomal proteins were found to account for 90% of the total ECM abundance, while 24 cellular proteins were still retained, accounting for 10% of the abundance. Sub-classification on the basis of function showed that fibrillar collagen was conserved across all the three stages, while the number of secreted proteins significantly diminished.

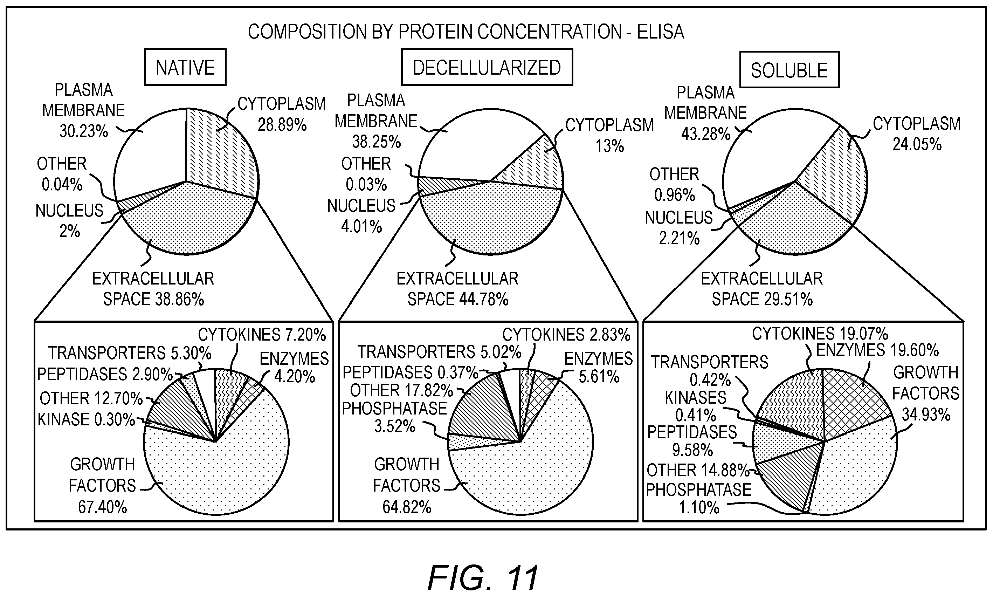

FIG. 11 presents a comparison of protein composition measured by ELISA of native, decellularized and soluble pancreas tissue. Only proteins that were identified in all the three batches were considered and classified on the basis of location for further analysis. Cellular proteins, mainly consisting of membrane, cytoplasmic and nuclear proteins accounted for 61% of the native proteome, while Extracelluar proteins accounted for ~39% of the total concentration. Similar to mass spec analysis, cellular proteins were removed from the assessment and only the extracelluar proteins was used for further investigation. They were sub-classified on the basis of protein type with growth factors and cytokines forming the two largest categories. A similar analysis and classification was performed on decellularized samples. ECM proteins were found to account for 45% of the total concentration the decellularized proteome, while the rest was cellular. ECM-only analysis revealed a high concentration of growth factors.

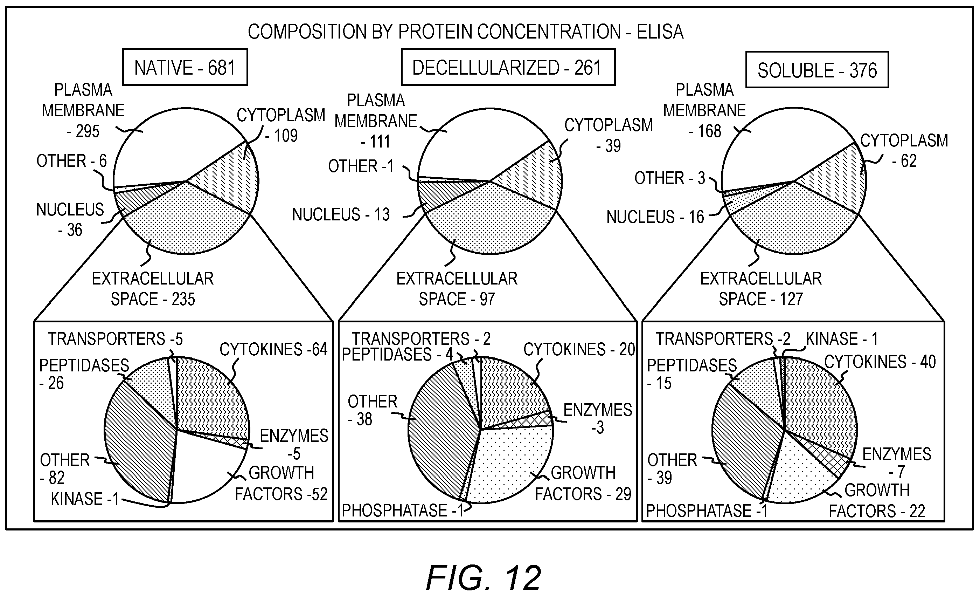

FIG. 12 presents a comparison based on number of proteins of native, decellularized and soluble pancreas tissue using ELISA. Classification on the basis of protein number revealed that 235 occupied the extracellular space, while 446 were cellular. After decellularization, many cellular proteins were still retained, while a loss in ECM proteins was also observed. The soluble ECM was composed of 376 proteins. Despite the difference in the number of proteins, the overall ratio of the various categories remained constant with decellularization and refinement. Soluble was found to be enriched in factors derived from both cellular and Extracellular parts. However, the % of cellular components had slightly increased.

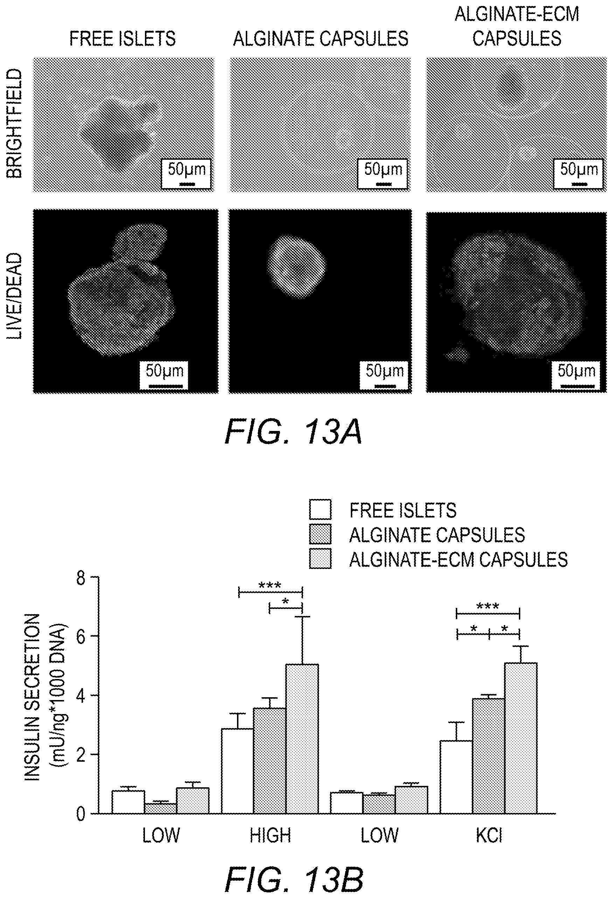

FIGS. 13A-13B presents results of culturing human islet cells with the pancreatic ECM prepared by the methods taught herein. FIG. 13A is brightfield and live/dead images of human isolated islets cultures as free, in alginate capsules and in alginate-ECM capsules at Day 6 post-encapsulation. FIG. 13B is a glucose stimulation assessment of human isolated islets cultured on non-tissue culture treated plates in three different settings: free, in alginate capsules, and in alginate-ECM capsules at Day 8 post-encapsulation. Values of the insulin secretion are reported after DNA normalization. Statistical comparisons of the insulin secretion are made between the three culture conditions in high glucose and after KCl depolarizing solution, respectively. *p < 0.05, ***p < 0.001.

### DETAILED DESCRIPTION OF EMBODIMENTS

Disclosed herein are methods of decellularizing tissues to form bioscaffolds and/or ECM compositions via a minimal processing method, as well as methods of use thereof.

The present invention is now described more fully hereinafter with reference to the accompanying drawings, in which embodiments of the invention are shown. This invention should not be construed as limited to the embodiments set forth herein; rather, these embodiments are provided so that this disclosure will be thorough and complete and will fully convey the scope of the invention to those skilled in the art.

As used herein in the description of the invention and the appended claims, the singular forms "a," "an" and "the" are intended to include the plural forms as well, unless the context clearly indicates otherwise. Furthermore, the terms "about" and "approximately" as used herein when referring to a measurable value such as an amount of a compound, dose, time, temperature, and the like, is meant to encompass variations of 20%, 10%, 5%, 1%, 0.5%, or even 0.1% of the specified amount. Also, as used herein, "and/or" refers to and encompasses any and all possible combinations of one or more of the associated listed items, as well as the lack of combinations when interpreted in the alternative ("or").

The transitional phrase "consisting essentially of" means that the scope of a claim is to be interpreted to encompass the specified materials or steps recited, and also additional materials or steps that do not materially affect the basic and novel characteristics of the claimed invention as described herein.

Unless otherwise defined, all terms (including technical and scientific terms) used herein have the same meaning as commonly understood by one of ordinary skill in the art to which this invention belongs. It will be further understood that terms, such as those defined in commonly-used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

"Bioscaffolds" or "scaffolds" as used herein refer to a substrate on which cells can grow. In preferred embodiments as taught herein, the bioscaffolds are derived from natural tissues. These scaffolds are useful in both the medical and research settings. For example, the bioscaffolds disclosed herein may be used in pancreatic, kidney, liver, or muscle tissue engineering and/or cell therapy, and/or may be used in cell culture including but not limited to 2-D or 3-D cell culture systems and bioreactor systems. The bioscaffolds may be formed, e.g., by rehydrating an ECM powder composition as taught herein to form a hydrogel.

"Natural tissues" are tissues that are normally found in an animal without human manipulation. Natural tissues that may be used to carry out the present invention may be isolated from any suitable animal source, including human, other mammalian (e.g., cat, dog, pig, cow, sheep, horse, monkey), avian (e.g., chicken, turkey, duck, goose, etc.), reptile, amphibian, etc. Tissues may be of any suitable type, including but not limited to: blood vessel *(e.g.,* vein, artery), skeletal muscle, smooth muscle *(e.g.,* bladder), cardiac muscle or heart, mall intestine, large intestine, kidney, liver, and pancreas.

"Subjects" as used herein are preferably human, but also include other mammals (e.g., cat, dog, pig, cow, sheep, horse, monkey), birds (e.g., chicken, turkey, duck, goose, etc.), reptiles, amphibians, etc.

"Cell culture" is the growth or proliferation of cells *in vitro.* "Cell" or "cells" as used herein may be any type of eukaryotic or prokaryotic cell, without limitation. Mammalian cells (including mouse, rat, dog, cat, pig, monkey and human cells) are in some embodiments preferred, e.g., for tissue engineering applications. In some embodiments, cells are provided in or further include a liquid carrier. The liquid carrier can be in the form of a suspension, solution, or any other suitable form. Examples of suitable liquid carriers include, but are not limited to, water, aqueous solutions (e.g., phosphate buffer solution, citrate buffer solution, etc.), liquid media (e.g., modified Eagle's medium ("MEM"), Hanks' Balanced Salts, etc.), gels such as hydrogels, and so forth, and in some embodiments may also include additional ingredients as desired.

Cells may be obtained from established cultures, donors, biopsy, or a combination thereof. In some embodiments, cells are stem cells or progenitor cells. In some embodiments, cells are primary cells. In some embodiments, cells are passaged.

"Pancreatic cells" include those cells normally found in the pancreas, and include pancreatic islet cells, e.g., glucagon-synthesizing A (α) cells, insulin-producing B (β) cells, D (δ) cells, etc., and any combination thereof. Pancreatic islet cells cultured by the processes described herein are useful for, among other things, production of insulin, implantation into a subject to treat diabetes (including type I and type II diabetes), etc.

"Kidney cells" include those cells normally found in the kidney, and include interstitial cells (e.g., interstitial peritubular cells which secrete erythropoietin), endothelial cells, etc., or any combination thereof. Kidney cells cultured by the processes described herein are useful for, e.g., production of erythropoietin, implantation into a subject to treat anemia or other kidney disease, etc.

"Liver cells" include those cells normally found in the liver, and include hepatoblasts, hepatocytes, hepatic stellate cells, Kupffer cells, sinusoidal endothelial cells, etc., including any combination thereof. Livers cells cultured using the processes described herein are useful, among other things, for implantation into a subject to treat acute or chronic liver disease.

"Muscle cells" include those cells normally found in muscle tissue, including smooth muscle cells, cardiac muscle cells, skeletal muscle cells, and any combination thereof. Muscle cells cultured with the processes described herein are useful for, among other things, implantation into a subject to treat muscle injuries or defects, and/or promote muscle healing.

Cells may be "attachment-dependent" (proliferating only after adhesion to a suitable culture surface or substrate), "attachment-independent" (able to proliferate without the need to attach to a surface or substrate), or contain both types, and growth parameters may be adapted accordingly. For example, some animal cell types, such as lymphocytes, can grow in suspension, while others, including fibroblasts and epithelial and endothelial cells, are attachment-dependent and must attach to a surface and spread out in order to grow. Other cells can grow either in suspension or attached to a surface.

Cells also include cell strains or cell lines, as known in the art, which are typically derived from cells found naturally in tissues. Cell lines differ from cell strains in that they have exceeded the Hayflick's limit and have become immortalized. Cell lines include, but are not limited to, cell lines of the cell types listed above, e.g., the pancreatic cell line MIN6, the liver cell line HepG, cancer cell lines such as W549 cells, Jurkat cells, HEK293 cells, prostate cancer cell lines, breast cancer cells lines, cervical cancer cell lines (e.g., HeLa cells), mouse 3T3 cells, etc.

*Methods of Decellularizing Tissues.* Provided herein are methods of decellularizing a tissue. In some embodiments, the tissue is diced (i.e., tissue that is processed into smaller pieces or otherwise dissected such that decellularizing is not performed by perfusion of an organ/tissue through the natural vessels/channels). In preferred embodiments, the decellularizing is a minimal processing method that makes use of a hypoosmotic solution, and in some embodiments is detergent-free (i.e., the tissue is not exposed to detergent during any step of the decellularizing process). The decellularizing is protease-free (e.g., the tissue is not exposed to an exogenous protease such as trypsin or dispase during any step of the decellularization process, although, in some embodiments, a protease such as pepsin may be used in a post-decellularizing solubilization step). In some embodiments, the decellularizing is toxin-free (e.g., the tissue is not exposed to an exogenous toxin such as latrunculin A or latrunculin B during any step of the decellularization process).

With reference to the example flow diagram provided in FIG. 1, the tissue may be decellularized with water such as deionized water as the hypoosmotic solution, for a time sufficient to lyse the cells of the tissue (24 hours in this example). Following this step, the tissue may be incubated with an enzyme to digest nucleic acids remaining after lysis (24 hours in this example). Then a wash step may be performed to remove the enzyme. The wash step may be performed with water or other hypoosmotic solution, and may include buffer and/or chelator such as Tris/EDTA to deactivate the enzyme. The washing step may include multiple washes, such as a final wash with water, to provide the decellularized tissue. The decellularized tissue may be dehydrated by freeze drying/lyophilizing, and milled into a power form such as by cryomilling. To render the powder soluble in aqueous solution, a digestion step with a protease (pepsin in this example) may be performed to de-lipidize the material, followed by recovery of the soluble ECM powder by dehydration with freeze drying/lyophilization.

With reference to the example schematic provided in FIG. 3, (1) pancreas tissue may be provided from a subject/donor (though other tissues may be used). (2) The tissue may be dissected/diced into smaller pieces, and incubated in a hypoosmotic solution with mechanical agitation such as shaking/stirring to lyse cells of the tissue. (3) The tissue may then be incubated in an enzyme solution to digest nucleic acids with mechanical agitation. (4) The decellularized tissue may be washed to remove the enzyme and/or remaining cell debris, to produce the decellularized tissue, which is comprised of the remaining extracellular matrix (ECM). (5) The decellularized tissue is dehydrated by lyophilization, and (6) milled to form a powder of pancreatic ECM. The powder may be (7) aliquoted into vials before being (8) de-lipidized by protease treatment, which renders the ECM more soluble in aqueous solution. (9) The solubilized ECM may be recovered by dehydration and/or filtration, to provide an UltraPure Soluble Powder of pancreatic ECM.

The "hypoosmotic solution" has an osmolarity sufficient to lyse cells of the tissue (i.e., sufficiently below cellular osmolarity). In some embodiments, the hypoosmotic solution comprises, consists of or consists essentially of water (e.g., deionized water, distilled water or ultrapure distilled water, sterile and/or pyrogen free water, etc.)

In some embodiments, the tissue is incubated in the hypoosmotic solution for a time of from 12 to 24, 36 or 48 hours, optionally while undergoing mechanical agitation of the tissue (e.g., while on an orbital shaker, with stirring of the solution, etc.) to decellularize the tissue. In some embodiments, the tissue may be incubated in the hypoosmotic solution at a temperature of from 2 to 15 degrees Celsius (e.g., about 4 degrees Celsius).

The method includes incubating the tissue with DNase (e.g., for a time of from 2 to 8, 10 or 12 hours), which may be followed by incubation in a solution that deactivates the enzyme (e.g., TRIS in water to chelate MgCl, deactivating DNase) (e.g., for a time of from 8 to 24, 30 or 36 hours). The DNase may be, for example, from animal origin, or human recombinant DNase such as dornase alfa.

It will be appreciated that not all steps of the example methods need be performed in some embodiments. For example, in some embodiments, an enzyme digestion step may not be needed/performed when processing pancreatic tissue since it contains endogenous enzymes that may be released and aid in digestion of the cells upon lysing the cells with the hypoosmotic solution.

Additional steps may also be performed. For example, additional incubation(s) of the tissue in a hypoosmotic solution may be performed in some embodiments, for example after the enzyme digestion step(s), to further decellularize the tissue (e.g., for a time of from 12 to 24, 36 or 48 hours).

In some embodiments, the tissue may be disinfected prior to, during, and/or after the decellularizing, which may be done, for example, by incubation in a disinfecting solution, antibiotic solution, or both (e.g., iodine solution such as bentadine, an antibiotic such as gentamicin, combinations thereof, etc.).

***ECM compositions and uses.*** As noted above, in some embodiments the decellularized tissue produced may be dehydrated and/or de-lipidized to provide ECM compositions. As illustrated in FIG. 3, the dehydrated tissue may be milled to form a powder, which powder may be used as-is or further processed by being de-lipidized to form a powder soluble in aqueous media/solution, and/or filtered to further purify and/or sterilize the composition. Further methods of forming soluble ECM powder are provided in U.S. Patent No. 10,085,946 to Machluf et al.. *See also* Sackett et al., Extracellular matrix scaffold and hydrogel derived from decellularized and delipidized human pancreas. Sci Rep, 8(1), 10452 (2018).

The compositions obtained through the decellularization methods taught herein ("ECM compositions") have a variety of uses. In some embodiments, the compositions are used to support and/or promote viability and/or growth of cells in culture. The compositions may be provided, for example, on a cell culture substrate (e.g., as a coating thereon), optionally wherein the ECM composition is provided in the form of a gel (e.g., hydrogel) or a powder. "Substrates" include porous, particulate, and non-porous (i.e., smooth) surfaces. Substrates may be a synthetic or natural material. The substrate may be formed from any suitable material, including, but not limited to, organic polymers (including stable polymers and biodegradable or bioerodable polymers), natural materials (e.g., collagen), metals (e.g., platinum, gold, stainless steel, etc.) inorganic materials such as silicon, glass, etc., and composites thereof. For example, styrene beads may be coated with the ECM preparations. Coating of the substrate may be carried out by any suitable means, such as spray coating, dip coating, or the like.

The cell culture substrate may comprise, for example, polystyrene and/or polypropylene. The substrate may be, for example, a petri dish, a 2-well plate, 6-well plate, a 12-well plate, a 24-well plate, or a 96-well plate. The substrate may be, for example, an insert configured to be placed into a cell culture dish (e.g., to be placed in a petri dish, a 6-well plate, a 12-well plate, or a 24-well plate). The substrate may comprise, for example, polycarbonate or polyester.

In some embodiments, cells may be contacted to a cell culture substrate, wherein said cells adhere to the coating. Any suitable substrate (e.g., cultureware such as petri dishes, 2-6-, 12-, 24-, 96-, or 384-well plates, etc., as noted above) may be coated or treated with an ECM preparation as described herein to promote the adhesion and/or growth and/or differentiation of cells for cell culture.

In some embodiments, ECM compositions as taught herein, such as the solubilized compositions, may be used as an additive to cell culture media. Such media may comprise or be intended to be used with serum, or may be serum-free. "Media" as used herein may be any natural or artificial growth media (typically an aqueous liquid) that sustains the cells. Examples include, but are not limited to, an essential media or minimal essential media (MEM), or variations thereof such as Eagle's minimal essential medium (EMEM) and Dulbecco's modified Eagle medium (DMEM), as well as blood, blood serum, blood plasma, lymph fluid, etc., including synthetic mimics thereof. In some embodiments, the growth media includes a pH color indicator (e.g., phenol red).

ECM compositions may also be used in three-dimensional cell culture or tissue construct. "Three-dimensional tissue construct" and "organoid" are used interchangeably herein and, as used herein, refer to a composition of live cells, typically in a carrier media, arranged in a three-dimensional or multi-layered configuration (as opposed to a monolayer). Suitable carrier media include compositions of the present invention (e.g., hydrogels, such as cross-linked hydrogels, comprising an ECM composition prepared in accordance with the present invention). In some embodiments, an organoid may be about 100 µm or 200 µm to about 350 or 500 µm in diameter, such as, for example, about 100, 150, 200, 250, 300, 350, 400, 450, or 500 µm. The organoid may comprise about 1,500, 2,000, 5,000 to about 10,000, 25,000, or 50,000 cells in total or about 1,000, 5,000, 10,000, or 50,000 to about 75,000, 100,000, or 150,000 cells in total.

As illustrated in FIGS. 9-12, processing of the ECM powder by de-lipidation provides a more soluble powder but also results in further loss of matrisome protein. Therefore, in some embodiments, the decellularized ECM composition that has not been de-lipidized is used, which may be in combination with the soluble ECM composition. For example, the decellularized ECM may be hydrated to form a hydrogel and/or coated onto a substrate for cell culture, and soluble ECM may be provided in the media for cell culture. In this way, the overall number of ECM proteins present in the cell culture may be increased.

The ECM compositions as taught herein may be used in methods of differentiating stem cells or progenitor cells, such as pancreatic progenitor cells (e.g., pluripotent stem cell-derived pancreatic progenitor cells) into insulin-secreting pancreatic beta cells, wherein growing the pancreatic progenitor cell in the presence of the composition enhances the differentiation of the progenitor cells into beta-like cells. The pancreatic beta cells may be formed in islet-like clusters that are responsive to glucose.

The ECM compositions as taught herein according to some embodiments may have desirable properties such as the retention of ECM components while successfully removing the native cells. In some embodiments, the ECM composition has less than 100 ng or 50 ng of DNA per mg dry weight (i.e., weight of the ECM composition after water is removed, such as by lyophilizing). In some embodiments, the ECM composition comprises a total collagen content of from 20-40 micrograms per milligram dry weight of the composition. In some embodiments, the ECM composition comprises a glycosoaminoglycan (GAG) content of from 2-10 micrograms per milligram dry weight.

In some embodiments, the ECM composition comprises an elastin content of from 5-25 micrograms per milligram dry weight of the composition.

In some embodiments, the ECM composition comprises one or more (e.g., 2, 3, 4, 5, 6, 7, 8, 9, 10, 11, 12) of the growth factor/growth factor families listed in Table 1.

In some embodiments, the ECM composition comprises a content of growth factors of greater than about 30, 40, 50, or 60% of the total abundance of an extracelluar matrix (ECM) component of the decellularized proteome.

In some embodiments, the ECM composition comprises a content of matrisome proteins of greater than about 70, 75, 80, 85, or 90% of the total abundance of the decellularized proteome, and a content of cellular proteins of less than about 20, 15, 12 or 10% of the total abundance of the decellularized proteome.

In some embodiments, the ECM composition (e.g., powder composition) has an endotoxin concentration of less than 0.5 EU/ml.

*ECM **containing** capsules.* ECM compositions as taught herein may be incorporated into cell therapies such as with the use of encapsulation techniques. For example, ECM compositions as taught herein may be used in a microparticle comprising encapsulated cells as taught by U.S. Patent No. 10,085,946 to Machluf et al.. The cells may be primary cells isolated from tissue, a cell strain or cell line, or stem or progenitor cells. In some embodiments, the cells are pancreatic, liver, muscle or kidney cells.

Such capsules may be used for methods of treating disease in animals such as humans, for example treating Type I diabetes with encapsulated pancreatic cells using pancreas-derived ECM compositions as taught herein, wherein the cells produce insulin. Use of the ECM compositions produced with the decellularization methods taught herein can improve the viability and the functionality of the pancreatic islets when encapsulated with alginate as compared to alginate only.

The present invention is explained in greater detail in the following non-limiting Examples.

### EXAMPLES

### Example 1: Detergent Free Enzymatic Decellularization Method for Producing Extracellular Matrix

We developed and evaluated a new decellularization method for porcine and human pancreata using a non-conventional decellularization technique that is not chemical or detergent based.

Decellularization is a technique commonly used in tissue engineering and regenerative medicine in order to obtain the extracellular matrix (ECM) from specific tissues. The ECM is a collection of several different molecules produced by supporting cells that provide structural, biochemical and functional support to the surrounding cells.

We have previously demonstrated that the pancreatic ECM from porcine and human origin can sustain the *in vitro* functionality and viability of pancreatic islets. *See* Peloso et al., "The human pancreas as a source of pro-toerogenic extracellular matrix scaffold for a new generation bio-artificial endocrine pancreas," Ann Surg. 264(1):169-179 (2016).

The roles of the ECM include providing a substrate for the structural organization of cells, providing a scaffold for cell attachment, and providing molecular factors that support angiogenesis, the maintenance of cell viability, proliferation and cell phenotype. Conventional decellularization techniques use ionic or non-ionic detergents (e.g., Triton X-100, SDS), which effectively clear the tissues' cellular compartments, but also result in the loss of important structural and bio-regulatory components of the ECM.

In order to reduce the loss of important and fundamental ECM-properties, we developed a detergent-free enzymatic method that is effective for organ decellularization and pancreatic ECM isolation. In preliminary experiments, human pancreatic ECM (hpECM) proved to be non-toxic and to promote improvements in both the viability and functionality of murine islets, *in vitro.* The hpECM was used as a biosupportive substrate for islets culture, and islets showed improved functionality, as compared to traditional islet culture techniques. These improvements are likely a result of growth factors, adhesion molecules and cytokines contained within the high quality hpECM. The collagens, glycosaminoglycans, fibronectins, laminins, and other proteins retained during the novel decellularization process may provide bio-regulatory cues that support greater cell viability and function. These promising data indicate that detergent-free decellularization and ECM component preservation may serve to maximize the biosupportive properties of the pancreatic ECM.

The importance of the ECM component of the pancreatic islet microenvironment has been appreciated, as islet function and viability are both compromised following collagenase treatment of the pancreas during islet isolation, and the loss of the ECM may be one of the reasons underlying the sub-optimal performance of islet transplantation. Commercially available matrix extracts may provide cells with basic structural proteins that help to sustain viability, function, proliferation and phenotype. However, these extracts have not proven to be sufficiently effective, safe, or amenable to clinical translation in islet cell therapy.

The pancreatic ECM can be obtained from human and porcine tissues with our methods consistently and in a sterile fashion for laboratory and pre-clinical applications for islet culture and transplantation.

Major innovative aspects of this work include: (i) the use of a detergent-free decellullarization protocol for ECM extraction; (ii) the development of an biosupportive/bioregulatory substrate that can be used for the support of pancreatic islets; and (iii) the development of a stable, supportive, clinically translatable islet substrate appropriate for *in vitro* and *in vivo* applications (wherein in vivo treatment is not according to the invention as claimed).

Considering the enormous population of patients with Type I diabetes (T1D), the ability to maximize current laboratory techniques for islet culture and to maximize the therapeutic potential of islet transplantation with this improved pancreatic ECM composition is significant.

### Materials and Methods

Porcine pancreata from healthy commercial pigs (6 months old) were harvested and stored in ice-cold sterile phosphate buffer saline (PBS) at -20°C until further use.

Human pancreata procured for transplantation purposes and then discarded, or directly donated for research, were collected under sterile conditions and stored at -20°C until further use.

### Surgical preparation of the pancreata and decellularization

Frozen pancreata were thawed overnight. Organs were surgically dissected in order to remove extrapancreatic tissues and vascular pedicles. Pancreatic parenchyma was surgically prepared as approximately 1cm³ cubes and underwent the following incubations before decellularization:
1. Incubation for 10 minutes in a disinfection solution (10ml betadine in 1L water solution).
2. Incubation for 10 minutes in an antibiotic solution (5ml of 40 mg/ml gentamicin in 995ml of sterile water).

Following the disinfection and antibiotic treatment, pancreatic cubes underwent the following treatment to obtain decellularization:
1. Pancreatic cubes were placed in a sterile 1L bottle with 1L of deionized water and subjected to incubation on a refrigerated shaker at 4 °C at 200 rpm for 24 hours.
2. Pancreatic cubes were placed into a sterile 1L bottle containing an enzymatic solution (50mg of DNAse, 950ml of UltraPure Deionized Water, 38.7ml of TRIS Buffer, 9.7ml of Magnesium Chloride (MgCl₂)) and subjected to incubation on a heated shaker at 37 °C at 100 rpm for 6 hours.
3. Pancreatic cubes were placed in a sterile 1L bottle with 1L of TRIS-EDTA solution (985 ml deionized water, 7.6 ml of TRIS buffer, 6.8 ml of EDTA solution) and subjected to incubation on a refrigerated shaker at 4 °C at 200 rpm for 18 hours.
4. Pancreatic cubes were placed in a sterile 1L bottle with 1L of deionized water and subjected to incubation on a refrigerated shaker at 4 °C at 200 rpm for 24 hours for a final wash.

Pancreatic cubes are now considered decellularized; therefore, the tissue retrieved at this stage is considered the pancreatic ECM. Representative H&E and DAPI histological images shown in FIG. 2 of native human pancreas (a) and human pancreatic ECM (b) decellularized with the minimal processing method demonstrated a complete loss of nuclear structures compared to the native pancreatic tissue. DAPI panel demonstrated a complete loss of nuclei compared to the native pancreatic tissue.

Pancreatic ECM was stored in 50 ml falcon tubes at -80 °C for 24 hours before undergoing lyophilization and cryomilling steps.

### Lyophilization and cryomilling of pancreatic ECM

Stored frozen pancreatic ECM underwent a process of lyophilization with the freeze dry technique for a period of 7 days.

Dry pieces of pancreatic ECM were collected at day 8 and cryomilled to a fine powder.

### Solubilization for Cell Culture Media Supplement

Powdered pancreatic ECM was solubilized in Pepsin-HCl for 48 hrs to obtain a soluble ECM, followed by pH neutralization at 7.4 (irreversible inactivation of Pepsin). The solubilized ECM was then lyophilized to create a soluble ECM powder that may be used as an additive to cell culture media, hydrated to form an ECM-containing hydrogel, etc. The pancreatic hydrogel was seen to undergo a process of crosslinking upon re-equilibration of the pH and/or the rising of the temperature.

The formed UltraPure Soluble ECM powder had an endotoxin concentration of less than 0.5 EU/ml. The pancreatic hydrogel also had an endotoxin concentration of less than 0.5 EU/ml.

### Safety Assays performed:

Viability: MTS, Apoptosis - tested with W549, JURKAT, HEK293 cell lines at concentrations of 0.125, 0.25, 0.5, 1, and 2 mg/mL UltraPure Soluble ECM. There was a reduction in cell viability only in JURKAT and HEK293 at the 2 mg/mL concentration. The JURKAT cell line was used for apoptosis assay. No apopotosis was found at any of the concentrations tested.

Hemocompatibility: platelet activation, hemolysis, coagulation, complement activation - JURKAT and A549 cell lines were tested for reactive oxygen species (ROS) production at concentrations of 0.0625, 0.125, 0.25, 0.5, 1, and 2 mg/mL UltraPure Soluble ECM. No ROS production was found in A549 at any of the concentrations tested, and ROS production was found in JURKAT only at 2 mg/mL. UltraPure ECM powder did not induce hemolysis, and had no effect in prothrombin time or partial activated thromboplastin time at 0.1 and 1 mg/mL in coagulation assay, and prolongation in thrombin time at 1 mg/mL. UltraPure Soluble ECM powder did not induce platelet activation at concentrations of 0.125, 0.25, 0.5, 1, and 2 mg/mL.

Lymphocyte Activation - UltraPure Soluble ECM powder did not induce lymphocyte activation at concentrations of 0.125, 0.25, 0.5, 1, and 2 mg/mL.

Cytokine Production - UltraPure Soluble ECM powder induced production of IL-6 and IL-8 at 1 mg/mL concentration.

### Pancreatic ECM composition growth factors and biochemical characterization

Preliminary testing indicated that the ECM growth factors listed in Table 1 were present in the resulting UltraPure ECM powder produced from the human pancreatic tissue.

**TABLE 1: Pancreatic ECM Growth Factors**

| **Family** | **Growth Factors** |
|---|---|
| Insulin-like Growth Factors | IGF-1, IGFBPs |
| Nerve Growth Factors (NGFs) | (BDNF, b-NGF, GDNF, NT-3, NT-4) |
| Vascular Endothelial Growth Factors | EG-VEGF, VEGF, VEGF-D, VEGF R2, VEGF R3 |
| Bone Morphogenetic Proteins (BMPs) | BMP4, BMP5, BMP7 |
| Epidermal Growth Factors | EGF, EGF-R, HB-EGF |
| Transforming Growth Factors | TGFa, TFGb1, TGFb3 |
| Fibroblast Growth Factors | bFGF, FGF-4, FGF-7 |
| Platelet Derived Growth Factors | PDGF-AA |
| Stem Cell Growth Factor | SCF |
| Placental Growth Factor | PIGF |
| Hepatocyte Growth Factor | HGF |
| Growth Hormone | GH |

FIGS. 4A-4C present a comparison of the biochemical characterization of native pancreas, acellular pancreatic ECM and soluble pancreatic ECM. FIG. 4A confirms satisfactory removal of DNA in the acellular pancreas and in the soluble pancreatic ECM. FIGS. 4B and 4C show significant differences in glycosaminoglycans and collagen quantification in the native pancreas compared to the acellular and solubilized ECM. Statistical analysis was performed by the t-test of native vs Decelled pancreas and native vs soluble ECM; ****=p<0.0001;***p<0.001; **p<0.01.

ECM elastin content of decellularized human pancreas tissue prepared with the minimal processing methods of the present application as compared to native pancreas is shown in FIG. 5.

### Min6-hpECM encapsulation

Min6 cells were encapsulated in alginate with hpECM added to the culture medium. After 6 days of culture, it was shown that cells with the hpECM showed viability and greater proliferation compared to the Min6 cell encapsulated without ECM (FIG. 6).

### Cell culture with pancreatic ECM

Min 6 cells were cultured as a monolayer with pancreatic hpECM powder in the culture media. As show in FIG. 7A, Min6 cell viability as a function of powder concentration in the culture media indicated by MTT absorbance (OD) suggests that the hpECM powder is not toxic.

Min6 cell glucose-stimulated insulin release (GSIR) as a function of powder concentration in the culture media as shown in FIG. 7B indicated that the hpECM powder may improve basal insulin secretion.

Using murine islets, we found that islet function measured through GSIR was improved by hpECM addition to culture substrates, suggesting that the hpECM powder was beneficial for preserving islet function during prolonged *ex vivo* culture (FIG. 8).

### References

- Peloso, L. Urbani, P. Cravedi, R. Katari, P. Maghsoudlou, M.E. Fallas, V. Sordi, A. Citro, C. Purroy, G. Niu, J.P. McQuilling, S. Sittadjody, A.C. Farney, S.S. Iskandar, J.P. Zambon, J. Rogers, R.J. Stratta, E.C. Opara, L. Piemonti, C.M. Furdui, S. Soker, P. De Coppi, G. Orlando, The Human Pancreas as a Source of Protolerogenic Extracellular Matrix Scaffold for a New-generation Bioartificial Endocrine Pancreas, Ann Surg 264(1) (2016) 169-79.
- R.O. Hynes, The extracellular matrix: not just pretty fibrils, Science 326(5957) (2009) 1216-9.
- J.C. Stendahl, D.B. Kaufman, S.I. Stupp, Extracellular matrix in pancreatic islets: relevance to scaffold design and transplantation, Cell transplantation 18(1) (2009) 1-12.
- S.H. Mirmalek-Sani, G. Orlando, J.P. McQuilling, R. Pareta, D.L. Mack, M. Salvatori, A.C. Farney, R.J. Stratta, A. Atala, E.C. Opara, S. Soker, Porcine pancreas extracellular matrix as a platform for endocrine pancreas bioengineering, Biomaterials 34(22) (2013) 5488-95.
- E. Korpos, N. Kadri, R. Kappelhoff, J. Wegner, C.M. Overall, E. Weber, D. Holmberg, S. Cardell, L. Sorokin, The peri-islet basement membrane, a barrier to infiltrating leukocytes in type 1 diabetes in mouse and human, Diabetes 62(2) (2013) 531-42.
- H.F. Irving-Rodgers, F.J. Choong, K. Hummitzsch, C.R. Parish, R.J. Rodgers, C.J. Simeonovic, Pancreatic islet basement membrane loss and remodeling after mouse islet isolation and transplantation: impact for allograft rejection, Cell transplantation 23(1) (2014) 59-72.
- D. Chaimov, L. Baruch, S. Krishtul, I. Meivar-Levy, S. Ferber, M. Machluf, Innovative encapsulation platform based on pancreatic extracellular matrix achieve substantial insulin delivery, J Control Release 257 (2017) 91-101.
- A. Peloso, R. Katari, R. Tamburrini, J. Duisit, G. Orlando, Glycosaminoglycans as a measure of outcome of cell-on-scaffold seeding (decellularization) technology, Expert Rev Med Devic 13(12) (2016) 1067-1068.
- D.O. Freytes, J. Martin, S.S. Velankar, A.S. Lee, S.F. Badylak, Preparation and rheological characterization of a gel form of the porcine urinary bladder matrix, Biomaterials 29(11) (2008) 1630-7.
- Orlando G, Gianello P, Salvatori M, et al. Cell replacement strategies aiming at reconstitution of the beta cell compartment in type 1 diabetes. Diabetes. 2014; 63:1433-1444. [PubMed: 24757193]
- Orlando G, Farney A, Sullivan DC, et al. Production and implantation of renal extracellular matrix scaffolds from porcine kidneys as a platform for renal bioengineering investigations. Ann Surg. 2012; 256:363-370. [PubMed: 22691371]
- Orlando G, Booth CL, Wang Z, et al. Discarded human kidneys as a source of ECM scaffolds for kidney regeneration technologies. Biomaterials. 2013; 34:5915-5925. [PubMed: 23680364]
- Salvatori M, Peloso A, Zambon JP, et al. Extracellular Matrix Scaffold Technology for Bioartificial Pancreas Engineering: State of the Art and Future Challenges. J Diabetes Sci Technol. 2014; 8:159-169. [PubMed: 24876552]

### Example 2: Further Characterization of ECM produced from Detergent Free Enzymatic Decellularization Method

We have developed a new, water-based protocol that does not use harsh detergents for organ decellularization. The resulting decellularized ECM can be processed to a powder useful to form a hydrogel, and optionally further refined to develop a soluble medium additive.

The newly-developed decellularization method enabled us to consistently obtain a highly purified ECM powder from the human pancreas. DNA quantification tests confirmed a satisfactory cell clearance. Native, acellular, and soluble pancreatic ECM were biochemically characterized in order to assess the preservation of pancreatic molecular fingerprint. Acellular and soluble pancreatic ECM was determined to be acellular, DNA-free (DNA < 50 ng.mg-1 of dry tissue) with consistent preservation of collagen and glycosaminoglycans. DNA analysis performed as proof of successful decellularization demonstrated clearance of deoxyribonucleic acid in both acellular and soluble pancreatic ECM (from 4.56µg/mg ± 3.42 to 30.05ng/mg ± 22.89 for the acellular pancreas p=0.0001; from 4.56µg/mg ± 3.42 to 22.81ng/mg ± 11.31 for the solubilized pancreatic ECM).

To further characterize the resulting ECM, we define the molecular fingerprint of the human pancreatic proteome, which includes the composition of the 1) native, 2) decellularized and 3) soluble ECM, batch-to-batch variability associated with each stage and the proteins that are retained or lost after each stage of refinement. For this study, 15 human pancreata were pooled on the basis of BMI (28.25) to form 3 batches of 5 pancreata each. They were then subjected to proteomic analysis by both Mass Spectrometry and multiplex (Kiloplex) ELISA for global discovery and targeted analysis, respectively.

As the pancreata were batched prior to proteomics, only the proteins that were identified in all the three batches with at least two unique peptides were considered for further analysis. Cellular proteins accounted for 69% of the human pancreatic proteome, while core matrisomal and matrisome associated proteins accounted for 22% and 9% of the total abundance based on label-free quantification (LFQ) values, respectively. For further investigation of ECM complexity and abundance of ECM proteins, cellular proteins were removed from the assessment and focus was laid only upon the matrisomal proteins. The matrisome was further classified based on either function or subcategory. Function-based analysis revealed that the ECM was ~65% fibrillar collagen, ~26% secreted proteins, 3.5% structural ECM, and ~5% combined FACIT collagens, basement membrane, regulatory and other ECM proteins. Classification on the basis of subcategory showed that collagens formed the most dominant category accounting for 69% of the total matrisome, based on LFQ. ECM regulators and ECM-affiliated proteins were the other main subcategories accounting for ~18% and 11% of the ECM, while ECM glycoproteins, proteoglycans and secreted factors formed less than 2% of the matrisome.

Classification on the basis of protein number revealed that 67 proteins formed the native matrisome, while 546 were found to be cellular proteins.

A similar analysis and classification was performed on samples decellularized by our non detergent-based technique. A shown in FIG. 9, matrisomal proteins were found to account for 91% of the total abundance of the decellularized proteome. Even though the DNA content was <50 ng/mg, 62 cellular proteins were still retained, accounting for 9% of abundance. The top 5 cellular proteins Pancreatic triacylglycerol lipase, Chymotrypsin-C, cytoskeletal Keratin type I, Phospholipase A2 and carboxypeptidase A accounted for 6% (out of 9%) of the cellular abundance. ECM-only analysis again revealed high abundance of Fibrillar Collagen, which had been enriched to 91% of the matrisome. All the other functional categories were found to be diminished. A loss of 28 matrisomal proteins was also observed.

The decellularized ECM was further refined to develop the soluble ECM, comprising of 51 proteins. 27 Matrisomal proteins were found to account for 90% of the total ECM abundance, while 24 cellular proteins were still retained, accounting for 10% of abundance. Fibrillar Collagen was again found to be further enriched, now constituting ~97% of the total ECM abundance, with other categories being further diminished.

Higher number of overall as well as ECM proteins were detected by ELISA compared to MS in decellularized and soluble. MS showed a higher loss in the number of proteins in soluble compared to decellularized (40 vs 28), while the opposite was true for ELISA (108 vs 138). This speaks to both the proteomic nature of the two fractions as well as the type of protein that each technique is more biased towards detecting.

These findings support the use of the two biomaterial fractions, decellularized and soluble, for cell culture combinatorially, in the form of a hydrogel and medium additive, respectively. When combined, for example, the cells will have access to an additional ECM proteins that were lost in some of the processing. As expected, fewer distinct proteins were detected as decellularization progressed, dropping from 613 to 101 and then 51 protein identifications in the native, decellularized and soluble conditions.

As shown in FIG. 10, Classification on the basis of protein number revealed that 67 proteins formed the native Matrisome, while 546 were found to be cellular proteins. After decellularization, 62 cellular proteins were still retained. A loss of 28 matrisomal proteins was also observed. The soluble ECM was composed of 51 proteins. 27 Matrisomal proteins were found to account for 90% of the total ECM abundance, while 24 cellular proteins were still retained, accounting for 10% of the abundance. Sub-classification on the basis of function showed that fibrillar collagen was conserved across all the three stages, while the number of secreted proteins significantly diminished, which is further evident in the next figure (switch to next slide) when different categories are compared side-by-side on the basis of both protein abundance and number across all the three stages.

The number of protein identifications dramatically decreased from 613 to 101 after decellularization. Matrisome proteins were well retained during decellularization, comprising 39 of the 101 proteins and 91% of the total LFQ intensity.

As shown in FIG. 11, cellular proteins, mainly consisting of membrane, cytoplasmic and nuclear proteins, accounted for 61% of the native proteome, while extracelluar proteins accounted for ~39% of the total concentration. Similar to mass spec analysis, cellular proteins were removed from the assessment and only the extracelluar proteins was used for further investigation. They were subclassified on the basis of protein type with growth factors and cytokines forming the two largest categories. Again, only the proteins that were identified in all the three batches were considered and classified on the basis of location for further analysis.

A similar analysis and classification was performed on decellularized samples. ECM proteins were found to account for 45% of the total concentration the decellularized proteome, while the rest was cellular. ECM-only analysis revealed a high concentration of growth factors.

Interestingly, the soluble ECM showed a decrease in the % concentration of ECM proteins and enrichment of cellular. Among the ECM proteins, cytokines were enriched while other categories were diminished. These results are in contrast to Mass Spec where matrisomal proteins, in particular fibrillar collagen was enriched with each stage of refinement.

As shown in FIG. 12, Classification on the basis of protein number revealed that 235 occupied the extracellular space, while 446 were cellular.

After decellularization, many cellular proteins were still retained, while a loss in ECM proteins was also observed. The soluble ECM was composed of 376 proteins. Despite the difference in the number of proteins, the overall ratio of the various categories remained constant with decellularization and refinement. Soluble was found to be enriched in factors derived from both cellular and Extracellular parts. However, the % of cellular components had slightly increased.

Sub-classification of ECM-only proteins showed that the number of growth factors had reduced with refinement but the number of cytokines had increased, which was consistent with the protein concentration data.

### Example 3: Human Islets Encapsulation and Culture

Human pancreatic islets were purchased from Prodo Laboratories, Inc (Aliso Vejo, CA, USA). Upon arrival human pancreatic islets were cultured in non-tissue culture treated plates for 24 hours under standard condition. Human islets were then manipulated in order to test the effect of the ECM on islets functionality and viability with the following experimental groups:
- 1-: Free Islets
- 2-: Islets encapsulated in alginate
- 3-: Islets encapsulated in alginate-ECM

The above mentioned groups of islets were cultured *in vitro* under standard conditions for up to 8 days to assess the effect of the ECM on insulin functionality and islets viability.

Alginate obtained from Nova-Matrix (Sandvika, Norway) was prepared mixing 1.5% (w/v) with HBSS (H6648, Sigma) and stirred overnight at 4 °C, the molecular weight reported by the manufacturer was between 75-200 kDA and G/M ratio of ≤ 1. For the experimental group containing the ECM, alginate was solubilized in HBSS containing ECM at 0.1 mg/ml.

Human pancreatic islets were encapsulated in alginate and alginate-ECM according to a previously described protocol (Tendulkar et al., A scalable microfluidic device for the mass production of microencapsulated islets. Transplant Proc, 43(9), 3184-7 (2011)). Briefly, islets were gently mixed with alginate and alginate-ECM at a concentration of 0.1 mg/ml of ECM. The obtained suspension was pumped through a microfluidic encapsulation device with 0.2mL/min and with of 2.0 of flow rate and air pressure respectively. Upon production, the microcapsules were collected in 100mM of CaCl₂ bath with 10mM of HEPES in order to allow alginate crosslinking for 10 minutes. Encapsulated islets were washed with HBSS prior to being cultured under standard condition at 37C with 5% CO₂ with media provided by the Prodo Laboratories and changed every other day until the end of the experiment. At Day 8, a GSIS and DNA analysis were performed to assess the production of insulin and as a measure of viability respectively.

Brightfield images and Live/Dead staining of free and encapsulated islets were performed to assess cellular morphology and viability. Qualitative assessment of the images was performed in order to assess islets health. Parameters taken into consideration were the shape, border, integrity, diameter of the islets and the presence of single cells in culture.

On day 8 post-encapsulation Human pancreatic islets were collected and incubated with Kreb's buffer containing low and high glucose concentration followed by KCl depolarization solution, and the molarity of these solutions were 2.8, 16.8 and 25mM respectively. The glucose challenge was performed after modification of a protocol previously described (Fraker, The Role of Oxygen During In Vitro Culture and Immunoisolation of Islets of Langerhans (2011)). Sephadex G-10 beads were inserted in poly prep chromatography tubes (731-1550, Bio-Rad), free islets or encapsulated islets were placed in the middle portion of the beads. Glucose and depolarizing solution were pipetted into the chromatographic tubes described above and incubated for 1 hour. A pre-incubation period with low molarity glucose solution was performed followed by a serial of incubations with low, high, low and depolarizing glucose solution. Medium from each incubation phase was collected and stored at -80 °C for subsequent analysis. Following incubation and collection of the depolarizing solution, human pancreatic islets were incubated with 1 ml of DNA extraction buffer, which was then collected and stored until further analysis. Insulin content was measured from glucose and depolarizing solution with the Mercordia kit following manufacturer protocol. Insulin measurements were normalized according to the DNA content measured with the Invitrogen picogreen kit (P11496, Invitrogen).

Group comparisons refer to the same batch of human islets received from the Prodo Laboratories with n = 3 independent assessment conducted for each assay. Values are expressed as Mean ± SD. The statistical analyses were performed using GraphPad Software 8.0 (GraphPad Software, La Jolla, CA, USA). Statistical tests included the Mann-Whitney test for the assessment of decellularization and DNA remnant analyses; unpaired t-test was used for the assessment of the glycosaminoglycans and collagen between the native, the decellularized pancreas and the soluble ECM; 2-way ANOVA with post hoc Turkey's multiple comparisons for the Glucose Stimulation Test in the assessment of the islets stimulation at Day8. Statistical significance was considered at p < 0.05 with designation of *p < 0.05, **p<0.01, ***p < 0.001, ****p < 0.0001.

Islets cultured in non-tissue culture treated plates were viable after 8 days of encapsulation and culture. Live/dead staining showed that islets cultured in the three different conditions were viable; however, more dead cells were present when un-encapsulated (FIG. 13A). Encapsulated islets maintained their spherical shape with a well-rounded border, a stable diameter and almost no single cells in culture. At the time point analyzed, free islets showed a tendency to aggregate, to develop irregularities at the borders and at the shapes and to show a darker core suggestive of necrotic event.

Both free and encapsulated islets were found to be glucose responsive at the time point analyzed (FIG. 13B). Islets encapsulated in ECM-alginate showed a statistical increase in insulin secretion following high glucose stimulation and KCl depolarizing solution compared both to free and alginate-only encapsulated islets.

The ECM produced with the detergent-free decellularization method proved to have a positive impact on the viability and the functionality of the islets when encapsulated with alginate as compared to alginate only.

### Example 4: Pancreatic ECM enhances differentiation towards a beta cell fate

Currently adopted decellularization methods use chemical detergent-based solutions that remove many critical components of the ECM matrisome such as GAGs, which exacerbates the loss of important signaling mediators like growth factors and cytokines. The herein disclosed decellularization protocol avoids the extraction of important GAGs content and allows for the preservation of the molecular integrity of the ECM-based biomaterial. This ECM composition when used as a medium supplement shows properties in the maintenance of primary islets and differentiation of pluripotent stem cell (PSC)-derived pancreatic progenitor (PP) cells into Islet-like clusters (ILCs), responsive to glucose. Specifically, initial experiments with the ECM-supplemented medium have shown an increased yield of β-like cells in ILCs differentiated from PP cells. Furthermore, these ILCs were responsive to glucose challenge, compared to control.

Assuming that pro-pancreatic factors residing within the pancreatic ECM would be most potent on differentiating stem cells that are already committed to pancreatic fate, differentiating cultures of SR1423 were exposed to pancreatic extract from day 16 to day 32 of differentiation. On day 16 of differentiation, greater than 95% of differentiating cells express the pancreatic transcription factor Pdx1. Differentiating clusters that were exposed to the ECM, and non-exposed controls were fixed and stained for expression of Pdx1, insulin and glucagon. On day 32, no difference between the cultures regarding expression of Pdx1 was evident. However, more insulin-expressing cells were evident in the cultures exposed to the pancreatic ECM.

Furthermore, cultures exposed to the pancreatic ECM secreted more insulin in response to glucose concentration than control cultures (data not shown). Therefore, we established that factors within the soluble pancreatic ECM influenced the maturation of cells that already had pancreatic identity, favoring the formation of insulin-secreting beta-like cells.

These results confirm the use of the ECM compositions taught herein for a differentiation protocol that does not include sorting and re-aggregation of beta cells to form an islet cluster, which is the currently the most successful, published differentiation method (Veres et al., (2019). Charting cellular identity during human in vitro β-cell differentiation. Nature, 1).

The foregoing is illustrative of the present invention, and is not to be construed as limiting thereof. The invention is defined by the following claims.

## Claims

1. A method of decellularizing a human tissue or a porcine tissue, wherein said method comprises:
(a) providing the tissue;
(b) incubating the tissue in a hypoosmotic solution for a time of from 12 to 24, 36 or 48 hours; and
(c) incubating the tissue with DNase,
to thereby decellularize the tissue, wherein the method does not comprise incubating the tissue with a detergent, and the method is protease-free.

2. The method of claim 1, wherein each of the incubating steps are carried out with mechanical agitation of the tissue.

3. The method of claim 1 or claim 2, wherein the tissue is pancreas, kidney, liver or muscle tissue, preferably wherein the tissue is pancreas tissue.

4. The method of any preceding claim, wherein said hypoosmotic solution consists essentially of or consists of water.

5. The method of any preceding claim, wherein the method is toxin-free.

6. The method of any preceding claim, wherein the tissue is diced.

7. The method of any preceding claim, wherein said method further comprises:
(d) incubating the tissue in a solution that deactivates the DNase; and then
(e) incubating the tissue in a second hypoosmotic solution for a time of from 12 to 24, 36 or 48 hours,
optionally wherein one or more of the incubating steps (b), (d), and (e) are carried out at a temperature of from 2 to 15 degrees Celsius, and/or
optionally wherein the incubating step (c) is carried out at a temperature of from 25 to 40 degrees Celsius, and/or
optionally wherein the tissue of the providing step has been disinfected.

8. The method of any preceding claim, wherein the method further comprises dehydrating the tissue after the decellularizing, and optionally wherein the method further comprises milling the tissue after the dehydrating to form a powder, further optionally wherein the method further comprises de-lipidization after the decelluarizing, dehydrating and/or milling.

9. A composition comprising decellularized tissue produced by a process of any preceding claim, wherein the composition comprises less than 100 ng or 50 ng of DNA per mg dry weight, and wherein the composition comprises a total collagen content of from 20-40 micrograms per milligram dry weight of the composition, optionally wherein the composition comprises a glycosoaminoglycan (GAG) content of from 2-10 micrograms per milligram dry weight of the composition, and/or wherein the composition comprises an elastin content of from 5-25 micrograms per milligram dry weight of the composition.

10. The composition of claim 9, wherein the composition comprises one or more of the following growth factor/growth factor families:
Insulin-like Growth Factors such as IFT-1, IGFBPs;
Nerve Growth Factors (NGFs) such as BDNF, b-NGF, GDNF, NT-3, NT-4;
Vascular Endothelial Growth Factors such as EG-VEGF, VEGF, VEGF-D, VEGF R2, VEGF R3;
Bone Morphogenetic Proteins (BMPs) such as BMP4, BMP5, BMP7;
Epidermal Growth Factors such as EGF, EGF-R, HB-EGF;
Transforming Growth Factors such as TGFa, TFGb1, TGFb3;
Fibroblast Growth Factors such as bFGF, FGF-4, FGF-7;
Platelet Derived Growth Factors such as PDGF-AA;
Stem Cell Growth Factor such as SCF;
Placental Growth Factor such as PIGF;
Hepatocyte Growth Factor such as HGF; and
Growth Hormone such as GH,
and/or wherein the composition comprises a content of growth factors of greater than about 30, 40, 50, or 60% of the total abundance of an extracellular matrix (ECM) component of the decellularized proteome, and/or
wherein the composition comprises a content of matrisome proteins of greater than about 70, 75, 80, 85, or 90% of the total abundance of the decellularized proteome, and a content of cellular proteins of less than about 20, 15, 12 or 10% of the total abundance of the decellularized proteome, and/or
wherein said composition has an endotoxin concentration of less than 0.5 EU/ml.

11. A cell culture substrate comprising a coating comprising the composition of claim 9 or 10, optionally wherein the coating is in the form or a gel or a powder, optionally wherein said substrate comprises polystyrene or polypropylene, and optionally wherein said substrate is a petri dish, a 2-well plate, 6-well plate, a 12-well plate, a 24-well plate, or a 96-well plate,
or optionally wherein said substrate is an insert configured to be placed into a cell culture dish, optionally wherein said cell culture dish is a petri dish, a 6-well plate, a 12-well plate, or a 24-well plate, and optionally wherein said substrate comprises polycarbonate or polyester; or
a cell culture media comprising the composition of claim 9 or 10, wherein the media is optionally serum-free.

12. A method for growing cells *in vitro* comprising the steps of:
contacting said cells to a cell culture substrate of claim 11, wherein said cells adhere to said coating; and
growing said cells *in vitro* under conditions conducive to the proliferation of said cells, optionally wherein the cell culture substrate comprises a decellularized tissue composition that has not been de-lipidized, and wherein the growing is carried out with media comprising a decellularized tissue composition that has been de-lipidized,
and/or optionally wherein said cells are pancreatic, liver, muscle or kidney cells, or progenitor cells thereof,
or optionally wherein said cells are stem cells or progenitor cells, and optionally wherein said growing is performed under conditions conducive to the differentiation of said stem cells.

13. A microparticle comprising encapsulated live cells and the composition of claim 9 or 10, wherein the live cells are pancreatic islet cells, optionally encapsulated in alginate.

14. The microparticle of claim 13 for use in a method of treating Type I diabetes in a subject in need thereof.

15. A method of differentiating pancreatic progenitor cells into pancreatic beta cells, said method comprising growing the pancreatic progenitor cell in the presence of the composition of claim 9 or 10, optionally wherein the pancreatic beta cells are formed in islet-like clusters that are responsive to glucose.

## Patentansprüche

1. Ein Verfahren zum Dezellularisieren eines menschlichen Gewebes oder eines Schweinegewebes, wobei das Verfahren Folgendes beinhaltet:
(a) Bereitstellen des Gewebes;
(b) Inkubieren des Gewebes in einer hypoosmotischen Lösung für eine Zeit von 12 bis 24, 36 oder 48 Stunden; und
(c) Inkubieren des Gewebes mit DNase,
um dadurch das Gewebe zu dezellularisieren, wobei das Verfahren nicht das Inkubieren des Gewebes mit einem Detergens beinhaltet und das Verfahren proteasefrei ist.

2. Verfahren gemäß Anspruch 1, wobei jeder der Inkubierungsschritte unter mechanischer Bewegung des Gewebes ausgeführt wird.

3. Verfahren gemäß Anspruch 1 oder Anspruch 2, wobei das Gewebe Pankreas-, Nieren-, Leber- oder Muskelgewebe ist, wobei das Gewebe vorzugsweise Pankreasgewebe ist.

4. Verfahren gemäß einem der vorhergehenden Ansprüche, wobei die hypoosmotische Lösung im Wesentlichen aus Wasser besteht oder aus Wasser besteht.

5. Verfahren gemäß einem der vorhergehenden Ansprüche, wobei das Verfahren toxinfrei ist.

6. Verfahren gemäß einem der vorhergehenden Ansprüche, wobei das Gewebe gewürfelt ist.

7. Verfahren gemäß einem der vorhergehenden Ansprüche, wobei das Verfahren ferner Folgendes beinhaltet:
(d) Inkubieren des Gewebes in einer Lösung, die die DNase deaktiviert; und dann
(e) Inkubieren des Gewebes in einer zweiten hypoosmotischen Lösung für eine Zeit von 12 bis 24, 36 oder 48 Stunden,
wobei optional einer oder mehrere der Inkubierungsschritte (b), (d) und (e) bei einer Temperatur von 2 bis 15 Grad Celsius ausgeführt werden und/oder wobei optional der Inkubierungsschritt (c) bei einer Temperatur von 25 bis 40 Grad Celsius ausgeführt wird und/oder
wobei optional das Gewebe des Bereitstellungsschritts desinfiziert wurde.

8. Verfahren gemäß einem der vorhergehenden Ansprüche, wobei das Verfahren ferner das Dehydratisieren des Gewebes nach dem Dezellularisieren beinhaltet und wobei optional das Verfahren ferner das Mahlen des Gewebes nach dem Dehydratisieren beinhaltet, um ein Pulver zu bilden, wobei das Verfahren ferner optional ferner die Delipidisierung nach dem Dezellularisieren, Dehydratisieren und/oder Mahlen beinhaltet.

9. Eine Zusammensetzung, die dezellularisiertes Gewebe beinhaltet, das durch einen Prozess gemäß einem der vorhergehenden Ansprüche hergestellt wurde, wobei die Zusammensetzung weniger als 100 ng oder 50 ng DNA pro mg Trockengewicht beinhaltet und wobei die Zusammensetzung einen Gesamtkollagengehalt von 20-40 Mikrogramm pro Milligramm Trockengewicht der Zusammensetzung beinhaltet, wobei optional die Zusammensetzung einen Glykosaminoglykangehalt (GAG-Gehalt) von 2-10 Mikrogramm pro Milligramm Trockengewicht der Zusammensetzung beinhaltet und/oder wobei die Zusammensetzung einen Elastingehalt von 5-25 Mikrogramm pro Milligramm Trockengewicht der Zusammensetzung beinhaltet.

10. Zusammensetzung gemäß Anspruch 9, wobei die Zusammensetzung eine(n) oder mehrere der folgenden Wachstumsfaktoren/Wachstumsfaktorfamilien beinhaltet:
insulinähnliche Wachstumsfaktoren, wie etwa IFT-1, IGFBPs;
Nervenwachstumsfaktoren (NGFs), wie etwa BDNF, b-NGF, GDNF, NT-3, NT-4;
vaskuläre endotheliale Wachstumsfaktoren, wie etwa EG-VEGF, VEGF, VEGF-D, VEGF R2, VEGF R3;
knochenmorphogenetische Proteine (BMPs), wie etwa BMP4, BMP5, BMP7;
epidermale Wachstumsfaktoren, wie etwa EGF, EGF-R, HB-EGF;
transformierende Wachstumsfaktoren, wie etwa TGFa, TFGb1, TGFb3;
Fibroblasten-Wachstumsfaktoren, wie etwa bFGF, FGF-4, FGF-7;
aus Blutplättchen gewonnene Wachstumsfaktoren, wie etwa PDGF-AA;
Stammzellen-Wachstumsfaktor, wie etwa SCF;
Plazenta-Wachstumsfaktor, wie etwa PIGF;
Hepatozyten-Wachstumsfaktor, wie etwa HGF; und
Wachstumshormon, wie etwa GH,
und/oder wobei die Zusammensetzung einen Gehalt an Wachstumsfaktoren von mehr als etwa 30, 40, 50 oder 60 % der Gesamthäufigkeit einer Komponente der extrazellulären Matrix (ECM) des dezellularisierten Proteoms beinhaltet und/oder wobei die Zusammensetzung einen Gehalt an Matrisomenproteinen von mehr als etwa 70, 75, 80, 85 oder 90 % der Gesamthäufigkeit des dezellularisierten Proteoms und einen Gehalt an zellulären Proteinen von weniger als etwa 20, 15, 12 oder 10 % der Gesamthäufigkeit des dezellularisierten Proteoms beinhaltet und/oder wobei die Zusammensetzung eine Endotoxinkonzentration von weniger als 0,5 EU/ml aufweist.

11. Ein Zellkultursubstrat, beinhaltend eine Beschichtung, die die Zusammensetzung gemäß Anspruch 9 oder 10 beinhaltet, wobei optional die Beschichtung in Form eines Gels oder eines Pulvers vorliegt, wobei optional das Substrat Polystyrol oder Polypropylen beinhaltet und wobei optional das Substrat eine Petrischale, eine 2-Well-Platte, eine 6-Well-Platte, eine 12-Well-Platte, eine 24-Well-Platte oder eine 96-Well-Platte ist,
oder wobei optional das Substrat ein Einsatz ist, der konfiguriert ist, um in eine Zellkulturschale platziert zu werden, wobei optional die Zellkulturschale eine Petrischale, eine 6-Well-Platte, eine 12-Well-Platte oder eine 24-Well-Platte ist und wobei optional das Substrat Polycarbonat oder Polyester beinhaltet; oder
ein Zellkulturmedium, das die Zusammensetzung gemäß Anspruch 9 oder 10 beinhaltet, wobei optional das Medium serumfrei ist.

12. Ein Verfahren zum Züchten von Zellen *in vitro,* das die folgenden Schritte beinhaltet:
Inkontaktbringen der Zellen mit einem Zellkultursubstrat gemäß Anspruch 11, wobei die Zellen an der Beschichtung haften; und
Züchten der Zellen *in vitro* unter Bedingungen, die der Proliferation der Zellen förderlich sind, wobei optional das Zellkultursubstrat eine dezellularisierte Gewebezusammensetzung beinhaltet, die nicht delipidisiert wurde, und wobei das Züchten mit einem Medium ausgeführt wird, das eine dezellularisierte Gewebezusammensetzung beinhaltet, die delipidisiert wurde,
und/oder wobei optional die Zellen Pankreas-, Leber-, Muskel- oder Nierenzellen oder Vorläuferzellen davon sind,
oder wobei optional die Zellen Stammzellen oder Vorläuferzellen sind und wobei optional das Züchten unter Bedingungen durchgeführt wird, die der Differenzierung der Stammzellen förderlich sind.

13. Ein Mikropartikel, das eingekapselte lebende Zellen und die Zusammensetzung gemäß Anspruch 9 oder 10 beinhaltet, wobei die lebenden Zellen Pankreasinselzellen sind, die optional in Alginat eingekapselt sind.

14. Mikropartikel gemäß Anspruch 13 zur Verwendung in einem Verfahren zur Behandlung von Typ-I-Diabetes bei einem Subjekt, das dies benötigt.

15. Ein Verfahren zum Differenzieren von Pankreasvorläuferzellen zu Pankreas-Betazellen, wobei das Verfahren das Züchten der Pankreasvorläuferzelle in Gegenwart der Zusammensetzung gemäß Anspruch 9 oder 10 beinhaltet, wobei optional die Pankreas-Betazellen in inselartigen Clustern gebildet werden, die auf Glucose ansprechen.

## Revendications

1. Un procédé de décellularisation d'un tissu humain ou d'un tissu porcin, dans lequel ledit procédé comprend :
(a) la fourniture du tissu ;
(b) l'incubation du tissu dans une solution hypoosmotique pendant une durée allant de 12 à 24, 36 ou 48 heures ; et
(c) l'incubation du tissu avec de la DNase,
pour ainsi décellulariser le tissu, dans lequel le procédé ne comprend pas l'incubation du tissu avec un détergent, et le procédé est exempt de protéase.

2. Le procédé de la revendication 1, dans lequel chacune des étapes d'incubation est effectuée sous agitation mécanique du tissu.

3. Le procédé de la revendication 1 ou de la revendication 2, dans lequel le tissu est du tissu pancréatique, rénal, hépatique ou musculaire, de préférence dans lequel le tissu est du tissu pancréatique.

4. Le procédé de l'une quelconque des revendications précédentes, dans lequel ladite solution hypoosmotique consiste essentiellement en ou consiste en de l'eau.

5. Le procédé de l'une quelconque des revendications précédentes, dans lequel le procédé est exempt de toxine.

6. Le procédé de l'une quelconque des revendications précédentes, dans lequel le tissu est découpé en dés.

7. Le procédé de l'une quelconque des revendications précédentes, dans lequel ledit procédé comprend en outre :
(d) l'incubation du tissu dans une solution qui désactive la DNase ; puis
(e) l'incubation du tissu dans une deuxième solution hypoosmotique pendant une durée allant de 12 à 24, 36 ou 48 heures,
facultativement dans lequel une ou plusieurs des étapes d'incubation (b), (d), et (e) sont effectuées à une température allant de 2 à 15 degrés Celsius, et/ou facultativement dans lequel l'étape d'incubation (c) est effectuée à une température allant de 25 à 40 degrés Celsius, et/ou
facultativement dans lequel le tissu de l'étape de fourniture a été désinfecté.

8. Le procédé de l'une quelconque des revendications précédentes, dans lequel le procédé comprend en outre la déshydratation du tissu après la décellularisation, et facultativement dans lequel le procédé comprend en outre le broyage du tissu après la déshydratation pour former une poudre, en outre facultativement dans lequel le procédé comprend en outre la délipidisation après la décellularisation, la déshydratation et/ou le broyage.

9. Une composition comprenant un tissu décellularisé produit par un processus de l'une quelconque des revendications précédentes, dans laquelle la composition comprend moins de 100 ng ou 50 ng d'ADN par mg de poids sec, et dans laquelle la composition comprend une teneur totale en collagène allant de 20 à 40 microgrammes par milligramme de poids sec de la composition, facultativement dans laquelle la composition comprend une teneur en glycosaminoglycane (GAG) allant de 2 à 10 microgrammes par milligramme de poids sec de la composition, et/ou dans laquelle la composition comprend une teneur en élastine allant de 5 à 25 microgrammes par milligramme de poids sec de la composition.

10. La composition de la revendication 9, dans laquelle la composition comprend un ou plusieurs des facteurs de croissance/familles de facteurs de croissance suivants :
des facteurs de croissance analogues à l'insuline tels que l'IFT-1, les IGFBP ;
des facteurs de croissance nerveux (NGF) tels que le BDNF, le b-NGF, le GDNF, le NT-3, le NT-4 ;
des facteurs de croissance endothéliale vasculaire tels que l'EG-VEGF, le VEGF, le VEGF-D,
le VEGF R2, le VEGF R3 ;
des protéines morphogénétiques osseuses (BMP) telles que la BMP4, la BMP5, la BMP7 ;
des facteurs de croissance épidermique tels que l'EGF, l'EGF-R, le HB-EGF ;
des facteurs de croissance transformants tels que le TGFa, le TFGb 1, le TGFb3 ;
des facteurs de croissance des fibroblastes tels que le bFGF, le FGF-4, le FGF-7 ;
des facteurs de croissance dérivés des plaquettes tels que le PDGF-AA ;
un facteur de croissance des cellules souches tel que le SCF ;
un facteur de croissance placentaire tel que le PIGF ;
un facteur de croissance des hépatocytes tel que le HGF ; et
une hormone de croissance telle que la GH,
et/ou dans laquelle la composition comprend une teneur en facteurs de croissance supérieure à environ 30, 40, 50, ou 60 % de l'abondance totale d'un composant de matrice extracellulaire (ECM) du protéome décellularisé, et/ou dans laquelle la composition comprend une teneur en protéines matrisomes supérieure à environ 70, 75, 80, 85, ou 90 % de l'abondance totale du protéome décellularisé, et
une teneur en protéines cellulaires inférieure à environ 20, 15, 12 ou 10 % de l'abondance totale du protéome décellularisé, et/ou
dans laquelle ladite composition a une concentration en endotoxine inférieure à 0,5 EU/ml.

11. Un substrat de culture cellulaire comprenant un revêtement comprenant la composition de la revendication 9 ou de la revendication 10, facultativement dans lequel le revêtement est sous la forme d'un gel ou d'une poudre, facultativement dans lequel ledit substrat comprend du polystyrène ou du polypropylène, et facultativement dans lequel ledit substrat est une boîte de Pétri, une plaque à 2 puits, une plaque à 6 puits, une plaque à 12 puits, une plaque à 24 puits, ou une plaque à 96 puits,
ou facultativement dans lequel ledit substrat est un insert configuré pour être placé dans une boîte de culture cellulaire, facultativement dans lequel ladite boîte de culture cellulaire est une boîte de Pétri, une plaque à 6 puits, une plaque à 12 puits, ou une plaque à 24 puits, et facultativement dans lequel ledit substrat comprend du polycarbonate ou du polyester ; ou
un milieu de culture cellulaire comprenant la composition de la revendication 9 ou de la revendication 10, dans lequel le milieu est facultativement exempt de sérum.

12. Un procédé de croissance de cellules *in vitro* comprenant les étapes de :
la mise en contact desdites cellules avec un substrat de culture cellulaire de la revendication 11, dans lequel lesdites cellules adhèrent audit revêtement ; et
la croissance desdites cellules *in vitro* dans des conditions propices à la prolifération desdites cellules, facultativement dans lequel le substrat de culture cellulaire comprend une composition de tissu décellularisé qui n'a pas été délipidisée, et dans lequel la croissance est réalisée avec un milieu comprenant une composition de tissu décellularisé qui a été délipidisée,
et/ou facultativement dans lequel lesdites cellules sont des cellules pancréatiques, hépatiques, musculaires ou rénales, ou des cellules progénitrices de celles-ci,
ou facultativement dans lequel lesdites cellules sont des cellules souches ou des cellules progénitrices, et facultativement dans lequel ladite croissance est réalisée dans des conditions propices à la différenciation desdites cellules souches.

13. Une microparticule comprenant des cellules vivantes encapsulées et la composition de la revendication 9 ou de la revendication 10, dans laquelle les cellules vivantes sont des cellules d'îlots pancréatiques, facultativement encapsulées dans de l'alginate.

14. La microparticule de la revendication 13 destinée à être utilisée dans un procédé de traitement du diabète de type I chez un sujet en ayant besoin.

15. Un procédé de différenciation de cellules progénitrices pancréatiques en cellules bêta pancréatiques, ledit procédé comprenant la croissance de la cellule progénitrice pancréatique en présence de la composition de la revendication 9 ou de la revendication 10, facultativement dans lequel les cellules bêta pancréatiques sont formées en grappes de type îlot qui sont sensibles au glucose.
